# EUROPEAN PATENT SPECIFICATION

(11) **EP 1 360 957 B1**
(45) Date of publication and mention of the grant of the patent: **18.01.2012**
(21) Application number: 02703009.7
(22) Date of filing: 05.02.2002
(51) Int. Cl.: A61K 9/00, A61K 9/20, A23L 1/302, A23L 1/304, A23L 1/29

(54) **VITAMIN/MINERAL COMPLEX**
VITAMIN/MINERAL-KOMPLEX
COMPLEXE VITAMINO-MINERAL

(30) Priority: 14.02.2001 RU 2001104090
(43) Date of publication of application: 12.11.2003
(73) Proprietor: Lazarev, Mikhail Ivanovich, 123060 Moscow (RU); Yenileyev, Rustam Khalilovich, 123458 Moscow (RU)
(72) Inventor: Lazarev, Mikhail Ivanovich, 123060 Moscow (RU); Yenileyev, Rustam Khalilovich, 123458 Moscow (RU)
(74) Representative: Curtis, Philip Anthony
(86) International application number: PCT/RU2002/000034
(87) International publication number: WO 2002/064111

(56) References cited:
- WO-A-01/43571
- DE-A1- 4 420 159
- GB-A- 2 252 729
- RU-C1- 2 139 935
- RU-C1- 2 143 891
- RU-C2- 2 154 466
- VANNUCCHI H: "INTERACTION OF VITAMINS AND MINERALS" ARCHIVOS LATINOAMERICANOS DE NUTRICION, vol. 41, no. 1, 1991, pages 9-18, XP009033008 ISSN: 0004-0622
- BUCK W B ET AL: "Toxicology and adverse effects of mineral imbalance" CLINICAL TOXICOLOGY 1973, vol. 6, no. 3, 1973, pages 459-485, XP009033012
- 'Informatsiya po primeneniju drazhe 'DUOVIT' upakovka, nomer I data registratsii v RF:P-8-242' no. 008004, 15 November 1996, pages 1 - 3, XP002971209

## Description

### Field of invention

The invention relates to the food and pharmaceutical industries: to the production of vitamin-mineral complexes (**complexes**) and food supplements, used for the prevention and treatment of vitamin-mineral deficiency, hereinafter **'deficiency'**.

### Prior art

A complex "Complivit" is known which contains 19 components in a tablet - vitamins: A; E; C; B₁; B₆; B₁₂; riboflavin mononucleotide; nicotinamide; folic acid; rutin; lipoic acid; calcium pantothenate, and minerals: iron(II) sulphate heptahydrate; copper sulphate; calcium phosphate; cobalt sulphate heptahydrate; manganese(II) sulphate pentahydrate; zinc sulphate heptahydrate; disubstituted magnesium phosphate (the composition is shown on the packaging of the preparation, which is produced by OAO UFAVITA). A disadvantage of Complivit is that not all the vitamins and minerals listed are compatible with one another during long-term storage and on assimilation by the human body.

A complex "Duovit" is known, which consists of two differently-coloured dragées, which when taken separately over the course of 24 hours meet the daily requirement of the human body for vitamins and minerals. One of the dragées (red) contains 11 vitamins: A; D₃; C; PP; E; B₁; B₆; B₁₂; calcium pantothenate; folic acid, while the other (blue) contains 8 minerals: magnesium; calcium; phosphorus; iron; zinc; copper; manganese; molybdenum. Said dragées also include sugar, artificial colorants and natural aromatic additives (see the packaging of the preparation, produced by KRKA, for the list).

It is known from generally-available information sources that zinc reduces the assimilation of copper and competes for assimilation with iron and calcium, while calcium and iron reduce the assimilation of manganese, as a consequence of which the combination of the said minerals in one dragée presupposes an increase in the daily doses of these in order to meet the said daily requirement, which is a disadvantage of Duovit. Separating the complex into two compositions, one of which includes vitamins and the other minerals, is not sufficient.

Document WO 01/43571 discloses associations of tablets comprising different vitamins and minerals, chosen so that no antagonists are present.

In "Interaction of vitamins and minerals" from Vannucchi, Archivos Latinoamericanos de nutricion, vol. 41 (1991), the interaction between selected mineral and vitamins are discussed.

### Substance of the invention

The object of the invention is to improve the health of the population.

The task of the invention was to create, with allowance for the mutual influence of vitamins and minerals, a complex formed by compositions which when taken separately would meet the requirement of the human body for vitamins and minerals while minimising the doses taken of these.

The technical result of employing the invention now declared comprises: a) minimising the daily doses of vitamins and minerals consumed while meeting the daily requirement of the body for these; b) the convenience of using vitamin-mineral compositions which include vitamins and minerals which are incompatible from the point of view of assimilation by the body; c) reduction in the labour content of production of vitamin-mineral complexes which includes vitamins and minerals which interact with one another; d) increase in the shelf life of vitamin-mineral complexes which includes vitamins and minerals which interact with one another.

The effect of the invention now declared is comprised in assimilation by the human body of the components included in the various compositions of the complex, as a consequence of creating conditions for their synergistic, while excluding those for antagonistic, interaction. The substance of the invention is comprised in dividing the complex into three to five compositions of predetermined composition, expressed below by specific distinguishing features, and determined by the age and food consumption preferences of the user.

An arrangement, containing preparative forms, of a vitamin-mineral complex which includes vitamins and minerals which interact with one another in the course of production and/or storage, and/or which includes vitamins and minerals which compete for assimilation by the body, and/or which includes vitamins and minerals which weaken the action of one another at the physiological level is proposed, characterised in that it is made to consist of three to five preparative forms of differing composition, intended to be taken separately, with the object of preventing and/or reducing said interaction, competition and weakening effect.

The arrangement now declared may also be such that: said forms are made in the form of dragées, or tablets, or capsules; each of said forms is coloured its own colour, different to the colour of the other forms; it includes a blister pack; said forms are disposed in a blister pack such that forms of one colour are located in one row.

A composition is proposed of a vitamin-mineral complex which includes vitamins and minerals which interact with one another in the course of production and/or storage, and/or which includes vitamins and minerals which compete for assimilation by the body, and/or which includes vitamins and minerals which weaken the action of one another at the physiological level, characterised in that it is made to consist of three to five preparative forms of differing composition, intended to be taken separately, with the object of preventing and/or reducing said interaction, competition and weakening effect.

The declared composition may also be such that one of its forms includes, as a minimum, vitamin E and the mineral selenium, and another, as a minimum, vitamins K and D or only vitamin K and the mineral calcium, with one of the remainder including, as a minimum, the minerals iron and copper, and another the minerals zinc and manganese, while the complex may be made up of differing forms; the above-mentioned vitamins and minerals are included in only one of the forms of the composition; the forms contain auxiliary substances; the forms contain colorants as auxiliary substances.

A method is proposed for manufacturing a vitamin-mineral complex which includes vitamins and minerals which interact with one another in the course of production and/or storage, and/or which includes vitamins and minerals which compete for assimilation by the body, and/or which includes vitamins and minerals which weaken the action of one another at the physiological level, comprised in that different compositions, intended to be taken separately, are prepared, for which purpose the corresponding components, including colorants, are mixed in amounts in accordance with the formulation, after which solid preparative forms of differing colour are prepared, characterised in that three to five compositions, are prepared, in which vitamins and/or minerals which act synergistically and favourably influence one another are mixed in order to prevent and/or reduce said interaction, competition and weakening effect.

It should be noted that combined, e.g. oestrogen-progesterone, preparations which comprise complexes including several compositions of differing colour, the make-up of which includes different components, are known to the Applicant (M.D. Mashkovskii, Lekarstvennye sredstva [Medicinal drugs], fourteenth edition, vol. 2, Moscow, Novaya Volna, 2000, pp. 49-55). However, the number of compositions included in the make-up of such complexes, and also the material from which they are made, are determined not by the prevention of adverse interaction between the components at the chemical and physiological levels, and not by the combination of synergistic components in a single composition, but by the need for a stagewise effect on the human body, depending on the present state of that body, corresponding to specified blood biochemistry (particularly hormonal composition, etc.). It follows from the above that while features similar to those now claimed are employed in the known preparations, a technical result different to that indicated above is achieved, in which connection the introduction of such features into the complex now declared is not obvious to the specialist working in the field of pharmacology.

In particular, in identifying the distinguishing features of the arrangement, composition and method now declared, the inventors, analysing generally-available sources of information and having obtained experimental confirmation of the synergistic and antagonistic action of the components, analytically came to a conclusion regarding the requirements for the presence of various elements of the complex, which determine the structure of the latter, in other words regarding the structure of a complex composition, determined by the combination of substances, which found reflection in the said minimum number, three, of compositions of a complex which meets the requirements of a human body for vitamins and minerals. This is easily demonstrated by a specific example. We will consider the minerals iron, manganese and calcium which are essential to the human body. It was noted above that iron and calcium reduce the assimilation of manganese, as a consequence of which manganese must be transferred into a composition which does not contain the first two components, while calcium reduces the assimilation of iron (see V.Griffit, Vitaminy, travy, mineraly i pishchevye dobavki [W.Griffith, Vitamins, herbs, minerals and food supplements], Moscow, Izdatel'stvo Grand, 2000), so that the latter must also be separated. The result is that the complex must consist of a minimum of three compositions.

In view of the information presented in the "Prior art" section, and the fact that an excess of iron leads to a deficiency of copper, and an excess of manganese to a deficiency of magnesium (see A.V.Skal'nyi, Mikroelementozy cheloveka [Human microelementoses], Moscow, Nauchnyi mir, 1999), the inclusion of copper and magnesium in the complex presupposes further division of the complex into 4, 5 or more compositions, but practical tests carried out by the inventors showed that the use of more than five compositions makes it much more difficult to follow the sequence and timing for taking the complex, limiting its field of application in clinical practice. An upper limit of 5 for the number of compositions in the complex was thus obtained.

Based on said analysis and experiments, the inventors identified features relating to the entire combination of vitamins and minerals, found in a particular aggregate state and constituting the various compositions of the complex now declared - the material from which the elements of the complex are formed; and, based on said practice, features relating to the ingredient make-up and the parameters of use of the compositions, features of the method for producing them, and also the forms, mutual disposition and characteristics of the elements of the complex.

Information confirming the feasibility of the invention The complex now declared is intended to be taken over a set period of time, for example over 24 hours.

The complex now declared contains vitamins and minerals in the form of three to five compositions of incompatible components designed to be taken separately, for example at different times.

In particular, it may include in its make-up 23 components, divided into 3 compositions, which are intended to be taken over a period of 24 hours. In this case, the components may be divided in such a manner that one of them will contain vitamins B₁, B₃ and B₆, and also the minerals iron, copper, molybdenum and iodine, another vitamins K, H, B₂, B₅, B₆, B₉, B₁₂ and D₃ and the minerals chromium and calcium, and the last vitamins A, E and C and the minerals magnesium, manganese, selenium and zinc.

The compositions of vitamins and minerals may be produced by mechanical mixing of the components in the required amounts, in accordance with the formulation.

Calcium stearate (2%) and aerosil (3%) or additionally water (5%) and gelatin solution (1%) may be added to the resultant mixtures.

The following auxiliary substances may be used when producing the aforementioned solid forms: edible gelatin,, Nipagin, stearic acid, lactose, potato starch, talc, calcium stearate, aerosil. Food colorants such as E 128, Red 2G, E-129.a, Allura Red AC, E-131, Patent Blue V, E-133, Brilliant Blue FCF, etc., may also be used as auxiliary substances.

Each composition may be coloured in a different manner to the others, and they may be packaged in a blister pack such that solid forms of one colour are disposed in one row.

The material produced may be pressed or granulated in order to obtain solid preparative forms (dragées, tablets, etc.), and also, for example, metered and packaged in capsules.

As already noted in the section "Substance of the invention", for the reasons stated in it, a complex may also consist of four or five compositions. Thus, a first composition may include vitamins B₁, B₃ and B₆, and the minerals Fe and Mo, a second - the minerals Cu and I, a third - vitamins B₂, B₅, B₆, B₉, B₁₂, D₃, H and K and the minerals Ca and Cr, and a fourth - vitamins A, E and C and the minerals Mg, Mn, Se and Zn.

In the case of five compositions, a complex may be formed as follows. First composition - vitamins B₁, B₃ and B₆, and the minerals Fe and Mo, second - the minerals Cu and I, third - vitamins B₂, B₉, B₁₂, D₃ and K and the minerals Ca, P, Mg and Zn, fourth - vitamins B₅, B₆ and H and the mineral Cr, and fifth - vitamins A, E and C and the minerals Mn and Se.

Use of the invention now declared makes it possible to secure a technical result which is manifested as follows.
a) Minimisation of the doses of vitamins and minerals taken while meeting the requirements of the body for these. In fact, dividing among separate preparative forms vitamins and minerals which are incompatible from the point of view of assimilation by the body allows the degree of their assimilation by the body to be increased while reducing the total amount taken.

At the same time, it is known that vitamin E and selenium, and also vitamin K and calcium, act synergistically; vitamin D is necessary for the assimilation of calcium; copper facilitates the assimilation of iron, etc. Including in one composition components which act positively on one another also facilitates achievement of the stated result.

The said division allows the daily dosage of the various vitamins and mineral substances to be reduced by a factor of 1.5 to 2.5. It is evident that optimising the total consumption of vitamins and minerals (particularly reducing the doses of certain vitamins and minerals taken) will facilitate an improvement in the health of the population, which will be particularly clearly manifested among persons suffering from illnesses associated with metabolic disturbances.
b) Convenience of use of vitamin-mineral complexes which include vitamins and minerals which are incompatible from the point of view of assimilation by the body. Colouring the preparative forms in different colours facilitates correct usage of the preparation.

The aforesaid placement of differently coloured compositions in a single blister pack makes it easier to follow the sequence for taking the compositions of the complex (removal of a said preparative form assumes mechanical breakage of the blister pack foil, resulting in the release of a dragée, tablet, capsule, etc.)
c) Reduction in the labour content of production of vitamin-mineral complexes which include vitamins and minerals which interact with one another - there is no need to introduce a series of additional additives which in conventional complexes ensure the stability of compositions which include such vitamins and minerals. A 5 to 10% reduction in said labour content is anticipated in mass-production conditions.
d) An increase of 1.2 to 3 times in the shelf life of vitamin-mineral complexes which include vitamins and minerals which interact with one another, which naturally occurs due to the absence of such vitamins and minerals in the various compositions of the complex.

The quantitative estimates presented have been obtained by the Applicant when performing pioneering studies and are based on the results of biochemical analyses.

## Claims

1. A vitamin-mineral complex which includes vitamins and minerals which interact with one another in the course of production and/or storage, and/or which includes vitamins and minerals which compete for assimilation by the body, and/or which includes vitamins and minerals which weaken the action of one another at the physiological level, **characterised in that** the complex is made of three to five preparative forms of differing composition, intended to be taken separately, in order to prevent and/or reduce said interaction, competition and weakening effect, wherein one of the forms includes, as a minimum, vitamin E and the mineral selenium, and another form includes, as a minimum either: (a) the vitamins K and D and the mineral calcium; or (b) the vitamin K and the mineral calcium.

2. A complex according to claim 1, wherein said forms are made in the form of dragees, capsules or tablets.

3. A complex according to claim 1 or 2, wherein said forms are coloured so that each said separate form is differently coloured to the other separate dose forms.

4. A complex according to any preceding claim, wherein said forms are contained in a blister pack.

5. A complex according to claim 4, wherein said forms are disposed in said blister pack such that forms of one colour are located in one row.

6. .A, complex according to any preceding claim, wherein said separate forms contain auxiliary substances.

7. A complex according to claim 6, wherein said auxiliary substances include colourants.

8. A complex according to claim 1, wherein one of the remaining forms includes, as a minimum, the minerals iron and copper.

9. A complex according to claim 8, wherein another of the remaining forms includes, as a minimum, the minerals zinc and manganese.

10. A complex according to any one of claims 1 to 7, wherein a first dose form comprises vitamins B₁, B₃ and B₆, and the minerals iron, copper, molybdenum and iodine, a second dose form comprises vitamins K, H, B₂, B₅, B₆, B₉, B₁₂ and D₃ and the minerals chromium and calcium; and a third dose form comprises the vitamins A, E and C and the minerals magnesium, manganese, selenium and zinc.

11. A complex according to any one of claims 1 to 7, comprising four forms, a first dose form comprising vitamins B₁, B₃ and B₆, and the minerals Fe and Mo, a second dose form comprising the minerals Cu and I, a third dose form comprising vitamins B₂, B₅, B₆, B₉, B₁₂, D₃, H and K and the minerals Ca and Cr, and a fourth dose form comprising vitamins A, E and C and the minerals Mg, Mn, Se and Zn.

12. A complex according to any one of claims 1 to 7, comprising five dose forms, a first dose form comprising vitamins B₁, B₃ and B₆, and the minerals Fe and Mo, a second dose form comprising the minerals Cu and I, a third dose form comprising vitamins B₂, B₉, B₁₂, D₃ and K and the minerals Ca, P, Mg and Zn, a fourth dose form comprising vitamins B₅, B₆ and H and the mineral Cr, and a fifth dose form comprising vitamins A, E and C and the minerals Mn and Se.

## Patentansprüche

1. Vitamin-Mineral-Komplex, der Vitamine und Mineralien beinhaltet, die im Verlauf der Herstellung und/oder Lagerung miteinander wechselwirken, und/oder der Vitamine und Mineralien beinhaltet, die um die Aufnahme vom Körper konkurrieren, und/oder der Vitamine und Mineralien beinhaltet, die die Wirkung voneinander auf physiologischer Ebene abschwächen, **dadurch gekennzeichnet, dass** der Komplex aus drei bis fünf Präparatformen mit unterschiedlichen Zusammensetzungen besteht, die dazu vorgesehen sind, separat eingenommen zu werden, um den Wechselwirkungs-, Konkurrenz- und Abschwächungseffekt zu verhindern und/oder zu mindern, wobei eine der Formen mindestens Vitamin E und das Mineral Selen beinhaltet und eine andere Form mindestens entweder: (a) die Vitamine K und D und das Mineral Kalzium oder (b) das Vitamin K und das Mineral Kalzium beinhaltet.

2. Komplex nach Anspruch 1, wobei die Formen in der Form von Dragees, Kapseln oder Tabletten hergestellt sind.

3. Komplex nach Anspruch 1 oder 2, wobei die Formen gefärbt sind, so dass jede separate Form anders gefärbt ist als die anderen separaten Dosisformen.

4. Komplex nach einem der vorhergehenden Ansprüche, wobei die Formen in einer Blisterpackung enthalten sind.

5. Komplex nach Anspruch 4, wobei die Formen derart in der Blisterpackung angeordnet sind, dass Formen einer Farbe sich in einer Reihe befinden.

6. Komplex nach einem der vorhergehenden Ansprüche, wobei die separaten Formen Hilfsstoffe enthalten.

7. Komplex nach Anspruch 6, wobei die Hilfsstoffe Farbstoffe beinhalten.

8. Komplex nach Anspruch 1, wobei eine der restlichen Formen mindestens die Mineralien Eisen und Kupfer beinhaltet.

9. Komplex nach Anspruch 8, wobei eine andere der restlichen Formen mindestens die Mineralien Zink und Mangan beinhaltet.

10. Komplex nach einem der Ansprüche 1 bis 7, wobei eine erste Dosisform die Vitamine B₁, B₃ und B₆ und die Mineralien Eisen, Kupfer, Molybdän und Jod umfasst, eine zweite Dosisform die Vitamine K, H, B₂, B₅, B₆, B₉, B₁₂ und D₃ und die Mineralien Chrom und Kalzium umfasst und eine dritte Dosisform die Vitamine A, E und C und die Mineralien Magnesium, Mangan, Selen und Zink umfasst.

11. Komplex nach einem der Ansprüche 1 bis 7, der vier Formen umfasst, wobei eine erste Dosisform die Vitamine B₁, B₃ und B₆ und die Mineralien Fe und Mo umfasst, eine zweite Dosisform die Mineralien Cu und I umfasst, eine dritte Dosisform die Vitamine B₂, B₅, B₆, B₉, B₁₂, D₃, H und K und die Mineralien Ca und Cr umfasst und eine vierte Dosisform die Vitamine A, E und C und die Mineralien Mg, Mn, Se und Zn umfasst.

12. Komplex nach einem der Ansprüche 1 bis 7, der fünf Dosisformen umfasst, wobei eine erste Dosisform die Vitamine B₁, B₃ und B₆ und die Mineralien Fe und Mo umfasst, eine zweite Dosisform die Mineralien Cu und I umfasst, eine dritte Dosisform die Vitamine B₂, B₉, B₁₂, D₃ und K und die Mineralien Ca, P, Mg und Zn umfasst, eine vierte Dosisform die Vitamine B₅, B₆ und H und das Mineral Cr umfasst und eine fünfte Dosisform die Vitamine A, E und C und die Mineralien Mn und Se umfasst.

## Revendications

1. Complexe Vitamines-substances minérales qui comprend des vitamines et des substances minérales qui interagissent les unes avec les autres au cours de la production et/ou du stockage, et/ou qui comprend des vitamines et des substances minérales qui sont en compétition pour leur assimilation par l'organisme, et/ou qui comprend des vitamines et des substances minérales qui atténuent l'action des unes des autres au niveau physiologique, **caractérisé en ce que** le complexe est composé de trois à cinq formes de préparation de compositions différentes conçues pour être prises séparément afin de prévenir et/ou réduire ledit effet d'interaction, de compétition ou d'atténuation, où l'une des formes comprend au minimum la vitamine E et le sélénium comme substance minérale et une autre forme comprend au minimum : (a) soit les vitamines K et D et le calcium comme substance minérale ; soit (b) la vitamine K et le calcium comme substance minérale.

2. Complexe selon la revendication 1, où lesdites formes se présentent sous la forme de dragées, de capsules ou de comprimés.

3. Complexe selon la revendication 1 ou 2, où lesdites formes sont colorées de telle sorte que chacune desdites formes séparées a une couleur différente de celle des autres formes galéniques séparées.

4. Complexe selon l'une quelconque des revendications précédentes, où lesdites formes sont contenues dans une plaquette thermoformée.

5. Complexe selon la revendication 4, où lesdites formes sont disposées dans ladite plaquette thermoformée de façon à ce que les formes d'une couleur donnée soient situées sur une rangée.

6. Complexe selon l'une quelconque des revendications précédentes, où lesdites formes séparées contiennent des substances auxiliaires.

7. Complexe selon la revendication 6, où lesdites substances auxiliaires comprennent des colorants.

8. Complexe selon la revendication 1, où l'une des formes restantes comprend, au minimum, le fer et le cuivre comme substances minérales.

9. Complexe selon la revendication 8, où une autre des formes restantes comprend, au minimum, le zinc et le manganèse comme substances minérales.

10. Complexe selon l'une quelconque des revendications 1 à 7, où une première forme galénique comprend les vitamines B₁, B₃ et B₆ et le fer, le cuivre, le molybdène et l'iode comme substances minérales, une seconde forme galénique comprend les vitamines K, H, B₂, B₅, B₆, B₉, B₁₂ et D₃ et le chrome et le calcium comme substances minérales et une troisième forme galénique comprend les Vitamines A, E et C et le magnésium, le manganèse, le sélénium et le zinc comme substances minérales.

11. Complexe selon l'une quelconque des revendications 1 à 7 qui comprend quatre formes : une première forme galénique comprenant les vitamines B₁, B₃ et B₆ et le Fe et le Mo comme substances minérales, une seconde forme galénique comprenant le Cu et l'I comme substances minérales, une troisième forme galénique comprenant les vitamines B₂, B₅, B₆, B₉, B₁₂, D₃, H et K et le Ca et le Cr comme substances minérales et une quatrième forme galénique comprenant les vitamines A, E et C et le Mg, le Mn, le Se et le Zn comme substances minérales.

12. Complexe selon l'une quelconque des revendications 1 à 7 qui comprend cinq formes galéniques : une première forme galénique comprenant les vitamines B₁, B₃ et B₆ et le Fe et le Mo comme substances minérales, une seconde forme galénique comprenant le Cu et l'I comme substances minérales, une troisième forme galénique comprenant les vitamines B₂, B₉, B₁₂, D₃, et K et le Ca, le P, le Mg et le Zn comme substances minérales, une quatrième forme galénique comprenant les Vitamines B₅, B₆ et H et le Cr comme substance minérale, et une cinquième forme galénique comprenant les vitamines A, E et C et le Mn et le Se comme substances minérales.
